(19) **Europäisches Patentamt / European Patent Office / Office européen des brevets**

(11) **EP 4 692 756 A1**

(12) **EUROPEAN PATENT APPLICATION**
published in accordance with Art. 153(4) EPC

(43) Date of publication:
**11.02.2026 Bulletin 2026/07**

(21) Application number: **24780764.7**

(22) Date of filing: **29.03.2024**

(51) International Patent Classification (IPC):
*G01N 3/34* (2006.01)     *G01N 33/02* (2006.01)

(52) Cooperative Patent Classification (CPC):
**G01N 3/34; G01N 33/02**

(86) International application number:
**PCT/JP2024/012951**

(87) International publication number:
**WO 2024/204669 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 JP 2023058961**

(71) Applicant: **MEIJI CO., LTD**
**Chuo-ku**
**Tokyo 104-8306 (JP)**

(72) Inventors:
- **INOUE, Motoki**
  **Hachioji-shi, Tokyo 192-0919 (JP)**
- **KANDA, Reina**
  **Hachioji-shi, Tokyo 192-0919 (JP)**

(74) Representative: **TBK**
**Bavariaring 4-6**
**80336 München (DE)**

(54) **METHOD FOR EVALUATING OBJECT TO BE CHEWED, OBJECT TO BE CHEWED, METHOD FOR PRODUCING OBJECT TO BE CHEWED, SYSTEM FOR EVALUATING OBJECT TO BE CHEWED, PROGRAM, AND RECORDING MEDIUM**

(57)     Provided is a method of evaluating an object to be chewed, the method including: performing an iterative compression process on the object to be chewed; and performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the object to be chewed that has been subjected to the iterative compression process.

## Description

Technical Field

The present invention relates to an object-to-be-chewed evaluation method, an object to be chewed, a manufacturing method for an object to be chewed, an object-to-be-chewed evaluation system, a program, and a recording medium.

Specifically, the present invention relates to: an object-to-be-chewed evaluation method which enables evaluation of characteristics of an object to be chewed from a viewpoint of eating conditions that may differ for each eater; an object to be chewed; a manufacturing method for an object to be chewed; an object-to-be-chewed evaluation system; a program; and a recording medium.

Background Art

In regard to food evaluation methods, there are technologies for analyzing food crushing involved in chewing or the like (Patent Literatures 1 to 5).

Specifically, in Patent Literature 1, there is disclosed an evaluation method for mouthfeel which includes a first step of acquiring a breaking curve of a test food, a second step of obtaining a power spectrum of the breaking curve, and a third step of calculating a specific peak in a desired frequency domain of the power spectrum as a mouthfeel evaluation value of the test food.

In Patent Literature 2, there is disclosed a method in which sounds and/or vibrations generated when a porous food is crushed or chewed are acoustically analyzed and a psychoacoustic evaluation value obtained by the acoustic analysis is used to evaluate mouthfeel of the porous food.

In Patent Literature 3, there is disclosed a method of evaluating a melt-in-the-mouth property of a food by measuring changes over time of a torque acting on a rotating portion that rotates under a state of being inserted into the food.

In Patent Literature 4, there is disclosed a method of evaluating mouthfeel based on a pressure distribution at a time of chewing.

In Patent Literature 5, there is disclosed a method of predicting mouthfeel by a regression analysis using a parameter relating to jaw movements exhibited when a subject chews a target food.

Citation List

Patent Literature

[PTL 1] JP 2001-133374 A
[PTL 2] JP 2017-26484 A
[PTL 3] JP 2021-85834 A
[PTL 4] JP 2020-134526 A
[PTL 5] JP 2022-66154 A

Summary of Invention

However, in the related-art technologies according to Patent Literatures 1 to 5, characteristics of an object to be chewed are not evaluated from a viewpoint of eating conditions that may differ for each eater, and it is not possible to evaluate a change in state of the object to be chewed that is being subjected to chewing, the state changing depending on the eating conditions.

One object of the present invention is to provide: an object-to-be-chewed evaluation method which enables evaluation of characteristics of an object to be chewed from a viewpoint of eating conditions that may differ for each eater; an object to be chewed; a manufacturing method for an object to be chewed; an object-to-be-chewed evaluation system; a program; and a recording medium.

Solution to Problem

As a result of extensive investigation, the inventors of the present invention have found that characteristics of an object to be chewed can be evaluated from a viewpoint of eating conditions that may differ for each eater by a regression analysis using, as explanatory variables, process condition parameters based on process conditions used for performing an iterative compression process on the object to be chewed, and have completed the present invention.

According to the present invention, it is possible to provide the following object-to-be-chewed evaluation method and the like.

1. A method of evaluating an object to be chewed, the method including:

performing an iterative compression process on the object to be chewed; and
performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the object to be chewed that has been subjected to the iterative compression process.

2. The method according to Item 1,

wherein the iterative compression process is performed through use of a processing device configured to be able to iteratively compress the object to be chewed, and
wherein the one or more process conditions include an operation condition of the processing device.

3. The method according to Item 1 or 2, wherein the one or more process condition parameters include a process condition parameter based on one or more selected from the group consisting of a compression force, a compression frequency, a compression speed, the number of times of compression, a rotation speed, a rotation angle, an artificial saliva addition amount, an artificial saliva composition, artificial saliva flow characteristics, a temperature, a clearance at a time of compression, a processing amount, and a compression plunger shape.

4. The method according to any one of Items 1 to 3, wherein the measurement parameter is based on one or more selected from the group consisting of: a load, an impulse, energy, an adhesion force, a torque, a displacement, and a strain that have been applied during the iterative compression process to the object to be chewed, a measurement value obtained by an acoustic sensor, a measurement value obtained by an olfactory sensor, a measurement value obtained by an optical sensor, a measurement value obtained by an electrochemical sensor, and changes over time of the measurement values; and a particle size distribution, hardness, adhesiveness, cohesiveness, a friction coefficient, a viscosity, and a moisture content of the object to be chewed that has been subjected to the iterative compression process, a measurement value obtained by the olfactory sensor, a measurement value obtained by the optical sensor, and a measurement value obtained by the electrochemical sensor.

5. The method according to any one of Items 1 to 4, wherein the object to be chewed is a solid matter or a semi-solid matter.

6. The method according to any one of Items 1 to 5, wherein the object to be chewed is a gummy candy.

7. The method according to any one of Items 1 to 6, wherein a regression equation obtained by the regression analysis is as follows:

$$Y = B_0 + B_1 X_1 + B_2 X_2 + \ldots + B_n X_n$$

where Y represents the response variable, $B_0$ represents a constant, $B_1$ to $B_n$ each represent a regression coefficient, $X_1$ to $X_n$ each represent one of the explanatory variables, and "n" represents an integer of 1 or more.

8. An object to be chewed, which is formed with an evaluation result obtained by the method of any one of Items 1 to 7 being set as target quality.

9. A manufacturing method for an object to be chewed, the manufacturing method including the method of any one of Items 1 to 7.

10. A system for evaluating an object to be chewed, the system including:

means for performing an iterative compression process on the object to be chewed; and
means for performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the

object to be chewed that has been subjected to the iterative compression process.

11. A program for causing a computer to operate each means of Item 10.

12. A computer-readable recording medium having recorded thereon the program of Item 11.

13. The recording medium according to Item 12, wherein the recording medium is a server on a network.

According to the present invention, it is possible to provide the object-to-be-chewed evaluation method which enables the evaluation of characteristics of an object to be chewed from the viewpoint of eating conditions that may differ for each eater; the object to be chewed; the manufacturing method for an object to be chewed; the object-to-be-chewed evaluation system; the program; and the recording medium.

Brief Description of Drawings

FIG. 1 is a schematic diagram for illustrating a configuration of an example of a processing device.
FIG. 2 is a schematic diagram for illustrating configurations of an upper plunger and a lower plunger of the processing device of FIG. 1;
FIG. 3 is a photograph of the upper plunger and the lower plunger of the processing device (1) of FIG. 1.
FIG. 4 is a graph for showing results of a regression analysis in Example 2.

Description of Embodiments

An object-to-be-chewed evaluation method, an object to be chewed, a manufacturing method for an object to be chewed, an object-to-be-chewed evaluation system, a program, and a recording medium according to the present invention are described in detail below.

The expression "x to y" as used herein represents the numerical range of "from x or more to y or less." An upper limit value and a lower limit value described for the numerical range may be freely combined.

In addition, two or more embodiments that are not contrary to each other out of the individual embodiments of an aspect according to the present invention to be described below may be combined, and an embodiment in which the two or more embodiments are combined is also an embodiment of the aspect according to the present invention.

1. Object-to-be-chewed Evaluation Method

An object-to-be-chewed evaluation method according to one aspect of the present invention is a method of evaluating an object to be chewed, the method including: performing an iterative compression process on the object to be chewed (hereinafter also referred to as "processing step"); and performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the object to be chewed that has been subjected to the iterative compression process (hereinafter also referred to as "analysis step").

According to the object-to-be-chewed evaluation method according to this aspect, characteristics of the object to be chewed can be evaluated from a viewpoint of eating conditions that may differ for each eater. Therefore, it is also possible to evaluate a change in state of the object to be chewed that is being subjected to chewing, the state changing depending on the eating conditions.

That is, while an act of eating may correspond to an act of performing the iterative compression process on the object to be chewed, this aspect involves performing the iterative compression process on the object to be chewed, and hence process conditions of the iterative compression process may thus correspond to the eating conditions. Therefore, the characteristics of the object to be chewed can be evaluated from the viewpoint of the eating conditions that may differ for each eater by a regression analysis using, as the explanatory variables, the process condition parameters based on the process conditions.

The characteristics of the object to be chewed include measurement values obtained during the iterative compression process from the object to be chewed and measurement values obtained from the object to be chewed that has been subjected to the iterative compression process. With a parameter corresponding to such characteristics of the object to be chewed being set as a response variable, it is possible to evaluate a degree of ease of eating depending on the eating conditions and a change in mouthfeel depending on the eating conditions.

(Processing Step)

In the processing step, the iterative compression process is performed on the object to be chewed.

There is no particular limitation on means for performing the iterative compression process on the object to be chewed.

In one embodiment, a processing device configured to be able to iteratively compress the object to be chewed is used for the iterative compression process. Through use of the processing device, various process conditions of the iterative compression process can be set with high accuracy and high reproducibility compared to when the chewing is performed by a human. In addition, through use of the processing device, process conditions that are difficult to be set when the chewing is performed by a human can also be set.

When the processing device is used, the above-mentioned process conditions can be set as operation conditions of the processing device. The term "iterative" means "one or more times," and the term "compression process" means a process for applying pressure to the object to be chewed to compress the object to be chewed.

The processing device is not particularly limited as long as the processing device is configured to be able to iteratively compress the object to be chewed. As the processing device, for example, a food physical property evaluation device as described in WO 2022/250167 A1 may be used.

For example, the processing device includes two or more members (plungers) for applying pressure to the object to be chewed, and is configured to be able to move one or more of the two or more members so that the object to be chewed can be compressed by being sandwiched between the two or more members and can be partially or completely decompressed.

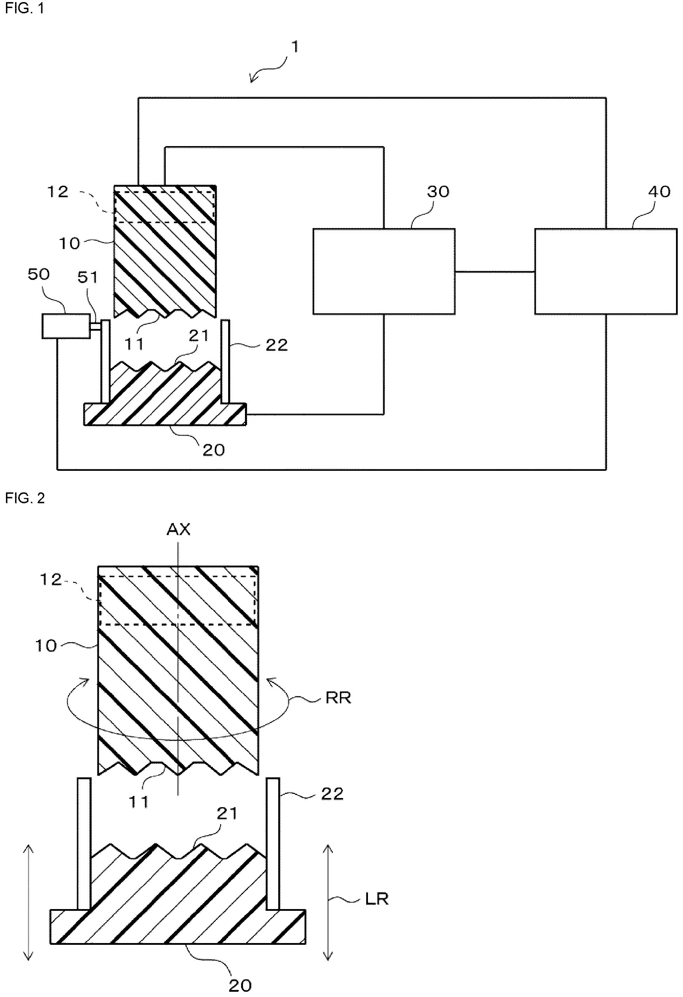

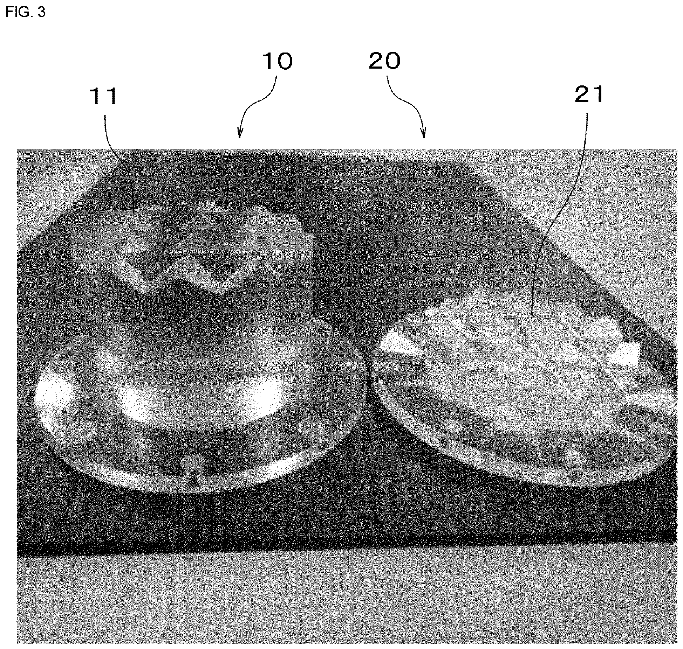

FIG. 1 is a schematic diagram for illustrating a configuration of the processing device in the one embodiment. A processing device 1 includes an upper plunger 10, a lower plunger 20, a sensor 12, a drive unit 30, and a measurement control unit 40. FIG. 2 is a schematic diagram for illustrating configurations of the upper plunger 10 and the lower plunger 20 of the processing device 1 of FIG. 1. FIG. 3 is a photograph of the upper plunger 10 and the lower plunger 20 of the processing device 1 of FIG. 1.

The upper plunger 10 includes an upper occlusal portion 11. The lower plunger 20 includes a lower occlusal portion 21 having a shape that occludes with the upper occlusal portion 11 so as to face the upper occlusal portion 11.

The lower occlusal portion 21 of the lower plunger 20 has a shape in which a plurality of quadrangular-pyramid-shaped protruding portions are arranged. The protruding portions of the lower occlusal portion 21 each have a quadrangular pyramid shape with a square base. The upper occlusal portion 11 of the upper plunger 10 has such an uneven shape as to occlude with the plurality of protruding portions of the lower occlusal portion 21.

The upper plunger 10 and the lower plunger 20 such as those illustrated and shown in FIG. 1 to FIG. 3 can be suitably used for evaluating, for example, characteristics of chocolate, a chocolate snack, chewing gum, a gummy candy, or the like.

The shapes of the upper plunger 10 and the lower plunger 20 are not limited to the example that is illustrated and shown, and any shape can be given depending on the type of the object to be chewed that is to be processed, the characteristics of the object to be chewed that is to be evaluated, and the like. As another example of the shapes of the upper plunger 10 and the lower plunger 20, the upper occlusal portion 11 of the upper plunger 10 may have, for example, a shape with a hemispherical protruding portion at a tip thereof, and the lower occlusal portion 21 may have a shape with a recessed portion having an inner wall surface being a hemispherical surface so as to occlude with the upper occlusal portion 11.

There is no particular limitation on a material of the upper plunger 10 and the lower plunger 20. It is preferred that the upper plunger 10 and the lower plunger 20 have a hardness suitable for forming an intraoral model. From such a viewpoint, a preferred material of the upper plunger 10 and the lower plunger 20 is a resin, for example: an acrylonitrile-butadiene-styrene copolymer (ABS) resin; an acrylic resin; a fluorine-containing resin such as polyvinylidene fluoride; a hard resin; and a dental resin.

Further, a material other than the resin may be used as the material of the upper plunger 10 and the lower plunger 20. Examples of the material other than the resin include metals and nonmetals. Examples of the metals include a gold-silver-palladium alloy, a gold alloy, and a platinum-added gold alloy. Examples of the nonmetals include ceramic and zirconia.

For example, through selection of a material that is used for dentures as the material of the upper plunger 10 and the lower plunger 20, application thereof to denture development (for example, evaluation of ease with which the object to be chewed sticks to dentures) and the like is also possible.

Each of the upper plunger 10 and the lower plunger 20 may be made of only one type of material, or may be made of a combination of two or more types of materials. For example, in the upper plunger 10 and the lower plunger 20 that are mainly formed of a first material, a part or entirety of a surface to be brought into contact with the object to be chewed may be formed of a second material. For example, the first material may be the above-mentioned resin, and the second material may be one or more types selected from the group consisting of the metals and the nonmetals described above. In this case, a method of applying the second material (for example, one or more types selected from the group consisting of the metals and the nonmetals) is not particularly limited, and, for example, a publicly-known surface coating method can be used without limitation, specifically, sputtering (vacuum deposition) or the like can be mentioned.

The sensor 12 is incorporated in the upper plunger 10, and measures a physical quantity applied to the upper plunger 10. The sensor 12 may be embedded in the upper plunger 10. The sensor 12 is, for example, a force sensor and a six-axis sensor. Examples of the physical quantity to be measured by the sensor 12 include a force and a torque applied to the upper plunger 10. The sensor 12 may also include, for example, a displacement sensor. The displacement sensor is configured to be able to measure moving distances of the upper plunger 10 and the lower plunger 20 or a deformation distance of the object to be chewed.

The drive unit 30 includes, for example, a motor. The drive unit 30 drives the lower plunger 20 so that the lower plunger 20 performs a reciprocating linear motion LR in a linear direction in which the lower plunger 20 occludes with and separates from the upper plunger 10. The drive unit 30 also drives the upper plunger 10 so that the upper plunger 10 performs a reciprocating rotational motion RR in a rotational direction with the linear direction of the reciprocating linear motion LR of the lower plunger 20 being set as a rotation axis AX.

The processing device 1 includes a pressure adjustment unit that adjusts pressure applied between the upper plunger 10 and the lower plunger 20 when the upper occlusal portion 11 of the upper plunger 10 and the lower occlusal portion 21 of the lower plunger 20 are brought into contact with each other at the time of occlusion. The pressure adjustment unit includes, for example, a solid elastic body such as a motor or a spring, and adjusts the pressure applied between the upper plunger 10 and the lower plunger 20 by a motive force of the motor or an elastic force of the solid elastic body. When the pressure adjustment unit is based on motor drive, the pressure adjustment unit may be driven by the motor that forms the drive unit 30. Means for adjusting the pressure applied between the upper plunger 10 and the lower plunger 20 is not limited to those described as examples, and may use, for example, air pressure. From a viewpoint of reducing hysteresis (history effect) and easily adjusting the pressure with high precision, it is preferred to use the solid elastic body such as a motor or a spring, in particular, a motor.

The measurement control unit 40 controls the reciprocating linear motion LR of the lower plunger 20 and the reciprocating rotational motion RR of the upper plunger 10 by the drive unit 30. The measurement control unit 40 also measures the physical quantity applied to the upper plunger 10 based on output from the sensor 12. The measurement control unit 40 can obtain data of an impulse by integrating data of the measured force with respect to time.

The processing device 1 obtains the physical quantity (measurement value) as the output from the sensor 12 when, with the object to be chewed that is to be evaluated being placed on the lower occlusal portion 21, the lower plunger 20 is driven so as to perform the reciprocating linear motion LR and the upper plunger 10 is driven so as to perform the reciprocating rotational motion RR.

In the processing device 1, for example, the upper plunger 10 and the lower plunger 20 are arranged at such positions as to prevent the upper plunger 10 and the lower plunger 20 from being brought into contact with each other even when the upper plunger 10 and the lower plunger 20 are closest to each other. The processing device 1 is configured to be able to adjust a clearance (also referred to as "clearance at a time of compression") between the upper plunger 10 and the lower plunger 20 when the upper plunger 10 and the lower plunger 20 are closest to each other.

When the object to be chewed that is to be evaluated is present on the lower occlusal portion 21, a force corresponding to a set occlusal force is applied from the lower plunger 20 to the object to be chewed, and is further applied to the upper plunger 10 through the object to be chewed. In the configuration, a force exceeding the set occlusal force is not applied during the occlusion between the upper plunger 10 and the lower plunger 20.

In the processing device 1, for example, the lower plunger 20 includes, on an outer periphery thereof, a cylindrical-shaped protective portion 22. The protective portion 22 prevents the object to be chewed from flying out from a space between the upper plunger 10 and the lower plunger 20 to the outside.

The processing device 1 further includes, for example, an artificial saliva supply unit 50 that adds artificial saliva and causes the artificial saliva to flow between the upper plunger 10 and the lower plunger 20 at a predetermined flow rate. An inflow tube 51 extends from the artificial saliva supply unit 50 completely through the protective portion 22 to the space between the upper plunger 10 and the lower plunger 20. In FIG. 1, only one inflow tube 51 is provided, but a plurality of inflow tubes may be provided. Under the control of the measurement control unit 40, the artificial saliva is added and caused to flow between the upper plunger 10 and the lower plunger 20 at the predetermined flow rate.

The artificial saliva is not always required to be supplied at the predetermined flow rate. For example, a predetermined amount of artificial saliva may be supplied between the upper plunger 10 and the lower plunger 20 at a start of a reciprocating compression process.

The artificial saliva is intended to reproduce saliva in an oral cavity, and is not particularly limited as long as the artificial saliva is a liquid, and examples thereof include water and an aqueous solution.

When an aqueous solution is used as the artificial saliva, components other than water in the aqueous solution are not particularly limited. For example, the aqueous solution may contain one or more kinds selected from the group consisting of: xanthan gum; sodium bicarbonate; dipotassium hydrogen phosphate trihydrate; sodium chloride; potassium chloride; calcium chloride dihydrate; mucin; a protein (albumin, globulin, lysozyme, lactoferrin, or histatin); a nitrogen compound (urea, uric acid, or creatinine); and an enzyme (amylase or maltase).

A composition and flow characteristics of the artificial saliva can be adjusted as appropriate. The flow characteristics (for example, a viscosity coefficient) of the artificial saliva can be adjusted by adjusting a concentration of a component (in particular, a component that changes the flow characteristics of water; for example, a thickening component such as mucin or xanthan gum) other than water in the aqueous solution. As the artificial saliva, a liquid having flow characteristics approximated to those of saliva can be used.

In the one embodiment, the artificial saliva is an aqueous solution of xanthan gum.

In the one embodiment, an aqueous solution which contains sodium bicarbonate, dipotassium hydrogen phosphate trihydrate, sodium chloride, potassium chloride, calcium chloride dihydrate, and mucin each in any amount and which is adjusted to pH 6.95 with hydrochloric acid can be used as the artificial saliva.

In other cases, in the one embodiment, those two kinds of aqueous solutions to each of which amylase is added can be used.

The processing device 1 is provided such that, for example, at least a part including the upper plunger 10 and the lower plunger 20 can be adjusted to a predetermined temperature. The predetermined temperature is, for example, a temperature close to a body temperature (30°C to 45°C). The part including the upper plunger 10 and the lower plunger 20 may be the entire processing device 1. The temperature can be adjusted through use of, for example, a hot air device or a heater.

The processing device 1 may include various sensors. Examples of the sensor include an acoustic sensor, an olfactory sensor, an optical sensor, and an electrochemical sensor.

The acoustic sensor may correspond to the sense of hearing of the eater. Through use of the acoustic sensor, it is possible to measure sounds (for example, volumes at respective frequencies) generated during the iterative compression process.

The olfactory sensor may correspond to the sense of smell of the eater. Through use of the olfactory sensor, it is possible to measure concentrations of various volatile substances evolved during the iterative compression process and after the iterative compression process.

The optical sensor can optically detect changes in states of the object to be chewed that is being subjected to the iterative compression process and that has been subjected to the iterative compression process. Through use of the optical sensor, it is possible to measure image information, the change in state of the object to be chewed, moisture distribution, temperature distribution, and eluted substances.

The electrochemical sensor may correspond to the sense of taste of the eater. Through use of the electrochemical sensor, it is possible to electrochemically measure the eluted substances during the iterative compression process and after the iterative compression process.

Through use of those sensors included in the processing device 1, various measurement values can be measured from the object to be chewed that is being subjected to the iterative compression process and that has been subjected to the iterative compression process.

A first example of movements (operation) of the processing device 1 described above is described below.

The object to be chewed that is to be evaluated is placed on the lower occlusal portion 21. Subsequently, the drive unit 30 drives the lower plunger 20 so that the lower plunger 20 performs the reciprocating linear motion LR in a direction in which the lower plunger 20 occludes with the upper plunger 10, and also drives the upper plunger 10 so that the upper plunger 10 performs the reciprocating rotational motion RR with the direction of the reciprocating linear motion LR of the lower plunger 20 being set as the rotation axis AX. When the upper occlusal portion 11 of the upper plunger 10 and the lower occlusal portion 21 of the lower plunger 20 are brought into contact with each other at the time of occlusion, the drive unit 30 adjusts the pressure applied between the upper plunger 10 and the lower plunger 20.

A specific example of the above-mentioned movements of the reciprocating linear motion LR of the lower plunger 20 and the reciprocating rotational motion RR of the upper plunger 10 is described.

First, the object to be chewed that is to be evaluated is placed on the lower occlusal portion 21 of the lower plunger 20. Subsequently, the lower plunger 20 is raised in a linear motion direction LR to cause the lower occlusal portion 21 of the lower plunger 20 to occlude with the upper occlusal portion 11 of the upper plunger 10.

Subsequently, when the lower occlusal portion 21 of the lower plunger 20 occludes with the upper occlusal portion 11 of the upper plunger 10, the object to be chewed is crushed by a predetermined force into the gap between the lower occlusal portion 21 and the upper occlusal portion 11. Under this state, the upper plunger 10 is rotated in a first rotational motion direction RR, to thereby subject the upper occlusal portion 11 of the upper plunger 10 to a sliding movement while being in contact with the object to be chewed.

Subsequently, the rotation of the upper plunger 10 in the first rotational motion direction RR is stopped, and the lower plunger 20 is lowered in the linear motion direction LR to release the occlusion between the lower occlusal portion 21 of the lower plunger 20 and the upper occlusal portion 11 of the upper plunger 10.

Subsequently, the lower plunger 20 is raised in the linear motion direction LR to cause the lower occlusal portion 21 of the lower plunger 20 to occlude with the upper occlusal portion 11 of the upper plunger 10. Under this state, the upper plunger 10 is rotated in a second rotational motion direction RR, which is the opposite direction to the first rotational motion direction RR, to thereby subject the upper occlusal portion 11 of the upper plunger 10 to a sliding movement while being in contact with the object to be chewed.

The above-mentioned movements are repeatedly performed. Of the above-mentioned movements, a step in which the lower plunger 20 is raised from the lowest position to occlude with the upper plunger 10 and is lowered back to return to the lowest position is counted as one as the number of times of compression.

A second example of the movements (operation) of the processing device 1 is described below.

A method of evaluating food physical properties through use of a food physical property evaluation device in this modification example is described. The object to be chewed that is to be evaluated is placed on the lower occlusal portion 21 of the lower plunger 20, and the lower plunger 20 is raised to cause the lower occlusal portion 21 of the lower plunger 20 to occlude with the upper occlusal portion 11 of the upper plunger 10. At this time, the upper plunger 10 and the lower plunger 20 are arranged at such positions as to prevent the upper plunger 10 and the lower plunger 20 from being brought into contact with each other even when the upper plunger 10 and the lower plunger 20 are closest to each other, and are caused to occlude with each other. When the lower occlusal portion 21 of the lower plunger 20 occludes with the upper occlusal portion 11 of the upper plunger 10, the object to be chewed is crushed by the predetermined force into the gap between the lower occlusal portion 21 and the upper occlusal portion 11. Subsequently, the lower plunger 20 is lowered to release the occlusion between the lower occlusal portion 21 of the lower plunger 20 and the upper occlusal portion 11 of the upper plunger 10. After the occlusion is released, the upper plunger 10 is rotated by 90 degrees in a horizontal direction until the upper plunger 10 reaches such a position as to prevent the upper plunger 10 and the lower plunger 20 from being brought into contact with each other even when the upper plunger 10 and the lower plunger 20 are closest to each other. The above-mentioned steps are repeated, to thereby perform the chewing a plurality of times. The 90-degree horizontal rotation of the upper plunger 10, which is performed for each time of chewing, is reversed, for example, every two times of chewing. The configurations and movements are the same, except those described above, as those in the above-mentioned first example of the movements (operation) of the processing device 1.

As the movements of the processing device 1, the movements described in the above-mentioned first example and second example may be combined as appropriate.

The movements of the processing device 1 can be appropriately set depending on the type of the object to be chewed, the purpose of the evaluation, and the like.

The processing device 1 is configured to be able to set one or more process conditions (operation conditions).

It is preferred that the processing device 1 be configured to be able to set one or more process conditions selected from the group consisting of, for example, a compression force, a compression speed, a compression frequency, the number of times of compression, a rotation speed, a rotation angle, an artificial saliva addition amount, an artificial saliva composition, artificial saliva flow characteristics, the temperature, the clearance at the time of compression, a processing amount, and a compression plunger shape.

The compressive force is a maximum force acting on the object to be chewed, and corresponds to an occlusal force at the time of chewing.

The compression speed is a compression distance per unit time, and corresponds to an occlusal speed at the time of chewing.

The compression frequency is the number of times of compression (occlusion) per unit time, and corresponds to an occlusal frequency at the time of chewing.

The number of times of compression corresponds to the number of times of occlusion at the time of chewing.

The rotation speed is an angular velocity to be exhibited when two members (for example, the upper plunger 10 and the lower plunger 20) are rotated, while the object to be chewed is compressed therebetween, relative to each other with a compression direction being set as a rotation axis. As a result of this relative rotation, the above-mentioned "sliding movement" can be performed. In regard to the relative rotation, one member is only required to rotate from the perspective of the other member, and with one member (for example, the lower plunger 20) fixed, the other member (for example, the upper plunger 10) may be rotated, while the two members may be rotated at mutually different angular velocities (in this case, a difference between the angular velocities of the two members can be set as the rotation speed). The rotation speed may be, for example, a speed (angular velocity) of the above-mentioned reciprocating rotational motion RR.

The rotation angle is an angle per relative rotation.

The artificial saliva addition amount corresponds to an amount of saliva at the time of chewing. The artificial saliva may be collectively added at a start of the iterative compression process, or may be continuously added during the iterative compression process. When the artificial saliva is added continuously, an artificial saliva addition flow rate can be set as the artificial saliva addition amount.

The artificial saliva composition can be expressed, for example, as a numerical value such as the concentration of a freely-selected component contained in the artificial saliva. The freely-selected component is not particularly limited, and when the artificial saliva is an aqueous solution, is particularly a component that changes the flow characteristics of water, examples of which include thickening components such as mucin and xanthan gum. The concentration of an enzyme such as amylase or maltase may also be used.

Examples of the artificial saliva flow characteristics may include the viscosity coefficient of the artificial saliva.

The processing amount is an amount of the object to be chewed that is to be supplied to the processing device for one iterative compression process, and corresponds to a morsel at the time of chewing.

The compression plunger shape includes shapes of two members (plungers) for compressing the object to be chewed, and includes, for example, the shapes of the upper plunger 10 and the lower plunger 20. Those shapes can be expressed, for example, as numerical values such as a height of each protruding portion, the number of protruding portions, a tip angle of each protruding portion, and a curvature of each protruding portion (the same applies to each recessed portion).

The processing device 1 can perform the movements of the reciprocating linear motion LR of the lower plunger 20 and the reciprocating rotational motion RR of the upper plunger 10, and simultaneously measure the physical quantity based on the output from the sensor 12.

In the processing device 1, an outer appearance of a food bolus during and after a predetermined number of times of chewing can be visually examined, and the food bolus can also be subjected to measurement of other physical properties. Further, a force acting on the upper plunger 10 at the time of occlusion and a torque acting on the upper plunger 10 due to rotational sliding between the upper plunger 10 and the lower plunger 20 are measured based on the output from the sensor 12. Further, the measurement control unit 40 obtains the data of an impulse by integrating data of the measured force (load) with respect to time. Further, changes over time of physical quantities, for example, changes over time of a force (impulse when integrated) and changes over time of torque, are obtained.

The iterative compression process using the processing device described above is described in detail.

The object to be chewed that is to be subjected to the iterative compression process (that is, the object to be chewed that is to be evaluated) is not particularly limited as long as the object to be chewed can be placed in the mouth and chewed therein by the eater. In this case, the eater may be a human or any animal (for example, a mammal such as a dog, a cat, a cow, a pig, a horse, and a rat) capable of chewing.

Further, the object to be chewed may be an object intended to be swallowed, or may be an object that is not intended to be swallowed.

Properties of the object to be chewed are not particularly limited, and are preferred to be, for example, a solid matter or a semi-solid matter. The "solid matter" refers to an object that is not in a flowable state at a normal temperature and a normal pressure. The "semi-solid matter" refers to an object in a state of being hardly deformed when no stress is applied at a normal temperature and a normal pressure but flowing when a slight stress is applied. The semi-solid matter may be, for example, in a paste-like, sauce-like, slurry-like, cream-like, or gel-like state, but is not limited thereto. In this case, the "normal temperature and normal pressure" mean a temperature of 25°C and 1 atmosphere (1,013.25 hPa).

The object to be chewed may be, for example, a food, a pharmaceutical, or a quasi-pharmaceutical product. As used herein, the terms "pharmaceutical" and "quasi-pharmaceutical product" are each defined in the Japanese "Act on Securing Quality, Efficacy and Safety of Products Including Pharmaceuticals and Medical Devices."

Examples of a form of the pharmaceutical include tablets, jelly forms, gummy candies, chewing gum, powders, and granules.

Examples of a form of the quasi-pharmaceutical product include tablets, jelly forms, gummy candies, chewing gum, powders, granules, hard candies, and paste forms.

Specific examples of the object to be chewed include gummy candies, chewing gum, chewing tobacco, chocolate, baked goods, cheese, meat products, fish products, egg products, soy products, substitute foods (for example, substitute meats), cultured foods (for example, cultured meats), and powdered or granular foods. In particular, for example, a gummy candy and chewing gum can be suitably used as the object to be chewed for an eating ability test described later.

For the iterative compression process, an object to be chewed that is to be evaluated is prepared. At this time, a plurality of the same objects to be chewed are prepared.

Subsequently, a predetermined number of (one or a plurality of) objects to be chewed among the prepared plurality of objects to be chewed are supplied to the processing device. The amount (or number) of object(s) to be chewed that is to be supplied to the processing device for one iterative compression process can be appropriately set depending on the size or the like of the object to be chewed. When a plurality of iterative compression processes are performed, it is preferred that this amount be constant. However, when the amount of the objects to be chewed that is to be supplied to the processing device for one iterative compression process is applied as a process condition to the process condition parameters, the amount can be changed for the plurality of iterative compression processes.

Subsequently, the object to be chewed that has been supplied to the processing device is subjected to the iterative compression process by the processing device under predetermined process conditions. The iterative compression process is a process for repeatedly compressing the object to be chewed, and can also be said to be a process for repeating compression and decompression of the object to be chewed. The iterative compression process can also be said to be a process that reproduces chewing movements.

The object to be chewed is usually compressed by reducing a distance between a pair of members (upper plunger and lower plunger) provided to the processing device and sandwiching the object to be chewed between the pair of members.

The object to be chewed is compressed, and is then decompressed. The decompression is usually achieved by increasing the distance between the pair of members. The decompression may be achieved by completely decompressing the object to be chewed (with the distance between the pair of members being larger than the size of the object to be chewed) or by partially decompressing the object to be chewed (with the distance between the pair of members being smaller than the size of the object to be chewed).

As the iterative compression process progresses, the state of the object to be chewed gradually changes. For example, the object to be chewed is gradually crushed and mixed with the artificial saliva. The object to be chewed crushed by the iterative compression process is also referred to as "food bolus." The food bolus may be, for example, the object to be chewed that has been crushed and mixed with the artificial saliva by the iterative compression process.

Subsequently, when a predetermined condition is satisfied, the iterative compression process is ended. The condition for ending the iterative compression process is not particularly limited, and a time point at which, for example, the number of times of compression, a duration of the iterative compression process, or an index of the crushing of the object to be chewed reaches a predetermined value can be set as an end point of the iterative compression process.

For example, a value based on measurement values of the force for respective times of compression can be applied as the index of the crushing of the object to be chewed. Specifically, for example, of maximum values of the measurement values of the force for the respective times of compression, an average value of the maximum values for the consecutive two times of compression can be applied. The time point at which this average value falls below the predetermined value can be set as the end point of the iterative compression process.

After the iterative compression process is ended, the food bolus may be removed from the processing device.

After the iterative compression process (first iterative compression process) is performed in the above-mentioned manner, the second iterative compression process is performed.

In the second iterative compression process, first, the same object to be chewed as in the first iterative compression process (as a new object to be chewed) is supplied to the processing device.

The second iterative compression process is performed in the same manner as the first iterative compression process except that the second iterative compression process is performed under a state in which one or more process conditions among the process conditions of the processing device used in the first iterative compression process are changed.

In this manner, the iterative compression process under one or more different process conditions is performed a plurality of times. The number of iterative compression processes does not mean the number of times of compression, but means the number of times that one set in which the object to be chewed is repeatedly compressed a predetermined number of times of compression (or until a predetermined condition is satisfied) is carried out.

The case in which a plurality of iterative compression processes are sequentially performed has been described above, but a plurality of processing devices may be used to simultaneously perform a plurality of iterative compression processes.

In each iterative compression process, one or more process conditions of the processing device and one or more measurement values selected from the group consisting of the measurement values obtained during the iterative compression process from the object to be chewed and the measurement values obtained from the object to be chewed that has been subjected to the iterative compression process are recorded.

(Process Condition)

The "process condition" of the iterative compression process may be any condition that can be quantified, and the "process condition" can be rephrased as "numerical value relating to the process condition." When the processing device is used for the iterative compression process, the process condition may be, for example, the operation condition of the processing device. The "operation condition of the processing device" can be rephrased as "setting value of the operation condition of the processing device."

Specific examples of the process condition include the compression force, the compression frequency, the compression speed, the number of times of compression, the rotation speed, the rotation angle, the artificial saliva addition amount, the artificial saliva composition, the artificial saliva flow characteristics, the temperature, the clearance at the time of compression, the processing amount, and the compression plunger shape. Details of those are as described above for the processing device.

(Measurement Value)

The measurement values obtained from the object to be chewed that is being subjected to the iterative compression process include a load, an impulse, energy, an adhesion force, a torque, a displacement, and a strain that have been applied during the iterative compression process to the object to be chewed, a measurement value obtained by the acoustic sensor, a measurement value obtained by the olfactory sensor, a measurement value obtained by the optical sensor, a measurement value obtained by the electrochemical sensor, and changes over time of those. Of those, details of the load, the impulse, the torque, the measurement value obtained by the acoustic sensor, the measurement value obtained by the olfactory sensor, the measurement value obtained by the optical sensor, and the measurement value obtained by the electrochemical sensor are as described above for the processing device.

The energy refers to a physical quantity indicating a magnitude of work expressed in units of N·m, and is the product of a force measured by the force sensor and a distance by which the object to be chewed is compressed.

The adhesion force refers to energy with a negative sign applied after a time point at which the upper plunger and the lower plunger are closest to each other until a time point at which the distance between the two plungers is largest in the iterative compression process, and is the product of the force measured by the force sensor and a distance by which the object to be chewed continues to adhere to the upper and lower plungers after the time point at which the upper plunger and the lower plunger are closest to each other until the time point at which the distance between the two plungers is largest in the iterative compression process.

The displacement refers to the moving distances of the upper and lower plungers or the deformation distance of the object to be chewed, and can be measured by the displacement sensor or by the displacement sensor and the force sensor.

The strain refers to a ratio between a length after deformation and a length before deformation, and can be measured by the displacement sensor and the force sensor.

The measurement values obtained from the object to be chewed that has been subjected to the iterative compression process include a particle size distribution, hardness, adhesiveness, cohesiveness, a friction coefficient, a viscosity, and a moisture content of the object to be chewed that has been subjected to the iterative compression process, the measurement value obtained by the olfactory sensor, the measurement value obtained by the optical sensor, and the measurement value obtained by the electrochemical sensor.

Of those, the details of the measurement value obtained by the olfactory sensor, the measurement value obtained by the optical sensor, and the measurement value obtained by the electrochemical sensor are as described above for the processing device.

The particle size distribution can be obtained by measuring a length, an area, a volume, or the like of particles, and a median value, an average value, or the like can be used as a representative value. For example, the particle volume can be measured by a three-dimensional shape measuring device, and the average value (average particle volume) can also be obtained from a result of the measurement.

The hardness, the adhesiveness, and the cohesiveness can be measured by a compression-tension rheometer.

The friction coefficient can be measured by a biaxial stress-controlled rheometer or the like.

The viscosity can be measured by a rotational rheometer or the like.

The moisture content can be measured by a heat-drying moisture meter or the like. When the moisture content is measured, the artificial saliva present in the food bolus can be appropriately removed by filtration or the like.

In each iterative compression process, a data set of one or more process conditions and one or more measurement values is obtained. A plurality of such data sets can be obtained by a plurality of iterative compression processes. Those plurality of data sets can be used to perform a regression analysis described later.

(Analysis Step)

In an analysis step, a regression analysis is performed based on data obtained by the iterative compression process described above (data set described above).

Specifically, in the analysis step, a regression analysis is performed through use of, as explanatory variables, one or more process condition parameters based on one or more process conditions of the processing device and, as a response variable, a measurement parameter based on one or more measurement values selected from the group consisting of a measurement value obtained during the iterative compression process from the object to be chewed and a measurement value obtained from the object to be chewed that has been subjected to the iterative compression process.

(Explanatory Variable)

The process condition parameter to be used as the explanatory variable is only required to be based on one or more process conditions, and may be, for example, one process condition itself or a function of one or more process conditions.

When the process condition parameter is a function of one process condition, examples of the function include a logarithm, a derivative, and an integral. The logarithm may be any one of a common logarithm (base=10), a natural logarithm (base=Napier's constant), or another logarithm.

When the process condition parameter is a function of two or more process conditions, examples of the function include the sum, the difference, the product, and the quotient of the two or more process conditions (or the logarithm thereof).

In the regression analysis, the number of explanatory variables may be one (simple linear regression analysis) or two or more (multiple regression analysis). When two or more explanatory variables are used, those explanatory variables are usually mutually independent variables.

When the number of explanatory variables is "n", the number of data sets (which can also be referred to as the number of iterative compression processes) required for the regression analysis is usually "n" or more.

(Response Variable)

The measurement parameter to be used as the response variable is only required to be based on one or more measurement values, and may be, for example, one measurement value itself or a function of one or more measurement values.

When the measurement parameter is a function of one measurement value, examples of the function include a logarithm. The logarithm may be any one of a common logarithm (base=10), a natural logarithm (base=Napierian constant), or another logarithm.

When the measurement parameter is a function of two or more measurement values, examples of the function include the sum, the difference, the product, and the quotient of the two or more measurement values (or the logarithm thereof).

(Regression Analysis and Regression Equation)

A regression equation obtained by the above-mentioned regression analysis is expressed, for example, by the following equation:

$$Y=B_0+B_1X_1+B_2X_2+...+B_nX_n$$

where Y represents the response variable, $B_0$ represents the constant, $B_1$ to $B_n$ each represent the regression coefficient, $X_1$ to $X_n$ each represent the explanatory variable, and "n" represents an integer of 1 or more.

A value of "n" is not particularly limited.

In the one embodiment, the value of "n" is 1 or more, 2 or more, or 3 or more, and is 20 or less, 15 or less, or 13 or less.

In a case of the simple linear regression analysis, n=1. The regression equation is expressed as $Y=B_0+B_1X_1$. In this case, it can be said that, as the absolute value of the regression coefficient $B_1$ becomes larger, an influence of the explanatory variable $X_1$ on the response variable Y becomes larger.

In a case of the multiple regression analysis, $n \geq 2$. In this case, it can be said that, as the absolute value of the regression coefficient B (also referred to as "partial regression coefficient B") becomes larger, an influence of the explanatory variable X corresponding to the regression coefficient B on the response variable Y becomes larger.

In addition, when standard partial regression coefficients $\beta_1$ to $\beta_n$ obtained by standardizing a plurality of regression coefficients $B_1$ to $B_n$ are used to compare the absolute values thereof, it is possible to compare the magnitudes of influences of explanatory variables $X_1$ to $X_n$ on the response variable Y.

In the one embodiment, the object-to-be-chewed evaluation method is carried out in the same manner for each of a plurality of types of objects to be chewed. In this case, the phrase "in the same manner" means that the same parameters are selected as the explanatory variable and the response variable. This enables comparison of the magnitude of an influence of a specific process condition parameter (specific eating condition) between a plurality of types of objects to be chewed, and enables relative evaluation of the characteristics of the objects to be chewed.

2. Object to be chewed

An object to be chewed according to one aspect of the present invention is an object to be chewed that is formed with an evaluation result obtained by the object-to-be-chewed evaluation method according to the one aspect of the present invention being set as target quality.

The object to be chewed according to this aspect can reflect the characteristics evaluated from the viewpoint of the eating conditions that may differ for each eater.

An object to be chewed according to another aspect of the present invention is an object to be chewed that is formed by being evaluated by the object-to-be-chewed evaluation method according to the one aspect of the present invention.

The characteristics of the object to be chewed according to this aspect have been evaluated from the viewpoint of the eating conditions that may differ for each eater, and hence the object to be chewed can be provided in accordance with the eating conditions of the eater.

The object to be chewed according to this aspect may be provided, for example, as a set of the object to be chewed and information based on the evaluation result obtained by the object-to-be-chewed evaluation method.

An object to be chewed according to further another aspect of the present invention satisfies the following regression equation which is obtained by the regression analysis in the object-to-be-chewed evaluation method according to the one aspect of the present invention:

$$Y=B_0+B_1X_1+B_2X_2+...+B_nX_n$$

where Y represents the response variable, $B_0$ represents the constant, $B_1$ to $B_n$ each represent the regression coefficient, $X_1$ to $X_n$ each represent the explanatory variable, and "n" represents the integer of 1 or more.

The value of "n" may be 1 or more, 2 or more, or 3 or more, and may be 20 or less, 15 or less, or 13 or less.

According to this aspect, for example, the target quality is set so that a specific regression coefficient (any one of $B_1$ to $B_n$) is large and the other regression coefficients are small, to thereby be able to predict a specific explanatory variable from the response variable without being influenced by other explanatory variables.

Further, the object to be chewed according to this aspect is known to satisfy the regression equation, and thus can be used for various purposes, for example, as the object to be chewed for the eating ability test described later.

The object to be chewed according to this aspect may be provided, for example, as a set of the object to be chewed and the regression equation (for example, information indicating the regression equation).

The object to be chewed according to each of those aspects of the present invention is further described by taking specific examples.

For example, it is possible to develop, based on a result of the regression analysis in the object-to-be-chewed evaluation method, an object to be chewed by setting the target quality so that a specific process condition parameter (specific eating condition) exerts a large influence on a specific measurement value parameter. The object to be chewed that has been obtained in this manner can be suitably used as an object to be chewed that serves to predict an eating ability of the eater. That is, the eater actually eats this object to be chewed, and then a measurement value corresponding to the specific measurement value parameter is obtained. It is possible to calculate the specific process condition parameter (specific eating condition) by substituting this measurement value into a regression equation obtained in advance as a result of the regression analysis in the object-to-be-chewed evaluation method. In this manner, the eating ability of the eater can be predicted (tested). In such a sense, the object to be chewed according to this aspect can also be said to be the object to be chewed for the eating ability test in the one embodiment.

As a specific example, in regard to the standard partial regression coefficients β obtained by the regression analysis using, as the explanatory variables, the process condition parameters based on the compression force, the compression speed, and the artificial saliva addition amount and, as the response variable, the particle size distribution (average particle volume) of the object to be chewed that has been subjected to the iterative compression process, it is possible to develop an object to be chewed (for example, gummy candy) in which the standard partial regression coefficient β for the artificial saliva addition amount is equal to or larger than a predetermined value and the standard partial regression coefficients β for the compression force and compression speed are equal to or smaller than the predetermined value. The object to be chewed that has been obtained in this manner can be used in a test for estimating a saliva flow rate of the eater from the particle size distribution (average particle volume) of the object to be chewed that has been subjected to the chewing by the eater.

As another specific example, a set of objects to be chewed that include a plurality of kinds of objects to be chewed can also be provided to test the eating ability of the eater from various angles. That is, when the set of objects to be chewed includes "m" kinds of objects to be chewed, it is possible to estimate "n" (n≤m) process condition parameters (eating abilities). For example, it is assumed that the same regression analysis has been used for each of the "m" kinds of objects to be chewed to obtain a regression equation between a response variable (measurement parameter, for example, the particle size distribution (average particle volume) of the object to be chewed that has been subjected to the chewing by the eater) and a plurality of explanatory variables (process condition parameters, for example, the compression force, compression speed, and the artificial saliva addition amount). At this time, for each of the "m" kinds of objects to be chewed, the particle size distribution (average particle volume) of the object to be chewed that has been subjected to the chewing by the eater is measured, and is substituted into each corresponding regression equation, to thereby obtain simultaneous equations including "m" regression equations. When a relationship of n≤m is satisfied, these simultaneous equations can be solved to obtain the values of the "n" process condition parameters. It is possible to evaluate, based on this result, the eating ability corresponding to each of the "n" process condition parameters.

Further, for example, it is possible to develop an object to be chewed so that an influence (absolute value of the regression coefficient corresponding to the saliva addition amount) of the process condition parameter (saliva addition amount) is smaller than a predetermined value in a regression equation obtained by a regression analysis using, as the explanatory variable, the process condition parameter based on the saliva addition amount and, as the response variable, the impulse applied to the object to be chewed by the processing device during the iterative compression process. The object to be chewed that has been obtained in this manner can provide an equivalent sense of chewiness regardless of a saliva amount (which may be influenced, for example, by aging, after exercise, or by side effects of medicine) of the eater.

3. Manufacturing Method for Object to be chewed

A manufacturing method for an object to be chewed according to one aspect of the present invention includes the object-to-be-chewed evaluation method according to the one aspect of the present invention.
According to the manufacturing method for an object to be chewed according to this aspect, the object to be chewed can reflect the characteristics evaluated from the viewpoint of the eating ability that may differ for each eater. That is, it is possible to manufacture, based on the result of the regression analysis in the object-to-be-chewed evaluation method, an object to be chewed in which a specific process condition parameter (specific eating condition) exerts a large influence on a specific measurement value parameter.

4. Object-to-be-chewed Evaluation System, Program, and Recording Medium

An object-to-be-chewed evaluation system according to one aspect of the present invention is a system for evaluating an object to be chewed, the system including: means for performing an iterative compression process on the object to be chewed; and means for performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the object to be chewed that has been subjected to the iterative compression process.
For the object-to-be-chewed evaluation system according to this aspect, the description given for the object-to-be-chewed evaluation method according to the one aspect of the present invention is incorporated herein by reference.
A program according to one aspect of the present invention is a program for causing a computer to operate each means of the object-to-be-chewed evaluation system according to the one aspect of the present invention.
A recording medium according to one aspect of the present invention is a computer-readable recording medium having recorded thereon the program according to the one aspect of the present invention.
According to the object-to-be-chewed evaluation system, the program, and the recording medium described above, the object-to-be-chewed evaluation method according to the one aspect of the present invention can be suitably carried out.
In the above description, the processing step and the analysis step in the object-to-be-chewed evaluation method is not required to be carried out as a series of steps. For example, information including at least one of the process condition or the measurement value in the processing step may be transmitted through a network such as the Internet, to thereby carry out the analysis step in a spatially different place. The same applies to respective means in the object-to-be-chewed evaluation system, and those are not required to be configured integrally. Further, the program is not required to be configured integrally, and can be configured as a plurality of mutually independent programs that operate respective means. Further, the recording medium is not required to be configured integrally, and can be configured as a plurality of recording media corresponding to the plurality of programs.
In the one embodiment, the above-mentioned recording medium is a server on a network.
For example, a program for operating means for performing the iterative compression process can be recorded on a server (recording medium) on the network. In this case, the user can operate the means for performing the iterative compression process based on the information received from the server through the network such as the Internet.
Further, for example, a program for operating means for performing the regression analysis can be recorded on the server (recording medium) on the network. In other words, an arithmetic processing unit that performs arithmetic operation processing of the regression analysis can be provided on the server. In this case, the user transmits the information including at least one of the process condition or the measurement value in the processing step from a terminal of the user to the server through the network such as the Internet. Then, the server performs the regression analysis based on the information received from the user. Then, the server transmits a result of the regression analysis to the terminal of the user. The result of the regression analysis may include one or more selected from the group consisting of the regression coefficient, the standard partial regression coefficient, and the regression equation.

It is also preferred that unique information on a food and result information on the regression analysis corresponding to the food be recorded on the recording medium as a database. In this case as well, the recording medium may be the server on the network. The unique information may be, for example, information for identifying the food, and may include, for example, a name of a manufacturer and a product name. The result information on the regression analysis may be, for example, one or more selected from the group consisting of the regression coefficient, the standard partial regression coefficient, and the regression equation. It is preferred that the recording medium have recorded thereon a plurality of information sets corresponding to a plurality of foods. The terminal of the user can acquire the plurality of information sets recorded on the recording medium. This allows the user to compare a plurality of foods from a viewpoint of analytical information.

Examples

Examples of the present invention are described below, but the present invention is not limited to those Examples.

(Example 1)

The objects to be chewed that were subjected to the evaluation by the object-to-be-chewed evaluation method are as follows.

- Object 1 to be chewed: "Fruit Juice Gummy Grape" manufactured by Meiji Co., Ltd.
- Object 2 to be chewed: "Cola Up" manufactured by Meiji Co., Ltd.
- Object 3 to be chewed: "Cola Up the Hard" manufactured by Meiji Co., Ltd.

As the processing device for the iterative compression process, the processing device 1 illustrated in FIG. 1 was used.

For each of the objects 1 to 3 to be chewed, the iterative compression process was carried out 54 times by varying the compression force, the compression frequency, and the number of times of compression as the operation conditions (process conditions of the iterative compression process) of the processing device. Specifically, the iterative compression process was carried out three times for each of total combinations (18 combinations) of three compression force conditions (200 N, 400 N, and 600 N), two compression frequency conditions (30 times/min and 60 times/min), and three number-of-times-of-compression conditions (20 times, 40 times, and 60 times). In those iterative compression processes, other process conditions were kept constant. The number of iterative compression processes (in this case, 54 times) does not mean the number of times of compression, but means the number of times that one set in which the object to be chewed is repeatedly compressed a predetermined number of times of compression (or until a predetermined condition is satisfied) is carried out. At the start of each iterative compression process, a new object to be chewed was subjected to the process.

After fine fragments from the object to be chewed that had been obtained after each iterative compression process (food bolus) were removed through use of a standard sieve having an opening of 1.40 mm, particles were arranged on a tray (200 mm×130 mm) so that the particles did not overlap each other, and the volume of each particle was measured through use of a three-dimensional shape measuring device ("VR-5000" manufactured by Keyence Corporation). An arithmetic mean of the measured volumes of the respective particles was set as the average particle volume.

The multiple regression analysis was performed through use of, as the explanatory variables, the compression force, the compression frequency, and the number of times of compression and, as the response variable, a logarithmic value of the average particle volume of the objects to be chewed that had been subjected to the iterative compression processes.

For the multiple regression analysis, "BellCurve for Excel ver. 3.2" manufactured by Social Survey Research Information Co., Ltd. was used.

The following values were obtained by the multiple regression analysis.

• Adjusted $R^2$

An adjusted $R^2$ is a coefficient of determination with adjusted degrees of freedom, and is an index indicating accuracy of the regression equation. The adjusted $R^2$ assumes a value of from 0 to 1, and as the value becomes closer to 1, the regression equation more closely fits actual data, which can be said to be more highly accurate.

• P-value of regression equation

A P-value of the regression equation is an index indicating the significance of the regression equation. When the significance level is set to 5%, a P-value smaller than 0.05 can be said to be significant.

• Standard partial regression coefficient $\beta$ for each of compression force, compression frequency, and number of times of compression

The standard partial regression coefficient $\beta$ is a partial regression coefficient standardized in order to compare influences of a plurality of partial regression coefficients on the response variable. A larger absolute value can be said to indicate a larger influence.

• P-value of partial regression coefficient for each of compression force, compression frequency, and number of times of compression

The P-value of the partial regression coefficient is an index indicating the significance of the explanatory variable corresponding to the partial regression coefficient. When the significance level is set to 5%, a P-value smaller than 0.05 can be said to be significant.

• Intercept of regression equation

Results of the above are shown in Table 1.

[Table 1]

|  | Adjusted $R^2$ | Regression equation P-value | Compression force | | Compression frequency | | Number of times of compression | | Intercept |
|---|---|---|---|---|---|---|---|---|---|
|  |  |  | $\beta$ | P-value | $\beta$ | P-value | $\beta$ | P-value |  |
| Object 1 to be chewed | 0.828 | < 0.05 | -0.146 | < 0.05 | 0.456 | < 0.05 | -0.780 | < 0.05 | 1.5860 |
| Object 2 to be chewed | 0.906 | < 0.05 | -0.193 | < 0.05 | 0.438 | < 0.05 | -0.826 | < 0.05 | 2.9295 |
| Object 3 to be chewed | 0.900 | < 0.05 | -0.138 | < 0.05 | 0.479 | < 0.05 | -0.811 | < 0.05 | 2.5509 |

The regression equations obtained for the objects 1 to 3 to be chewed are as follows. In the following equations, Y represents the logarithmic value of the average particle volume (mm$^3$), $X_1$ represents the compression force (N), $X_2$ represents the compression frequency (times/min), and $X_3$ represents the number of times of compression (times).

• Object 1 to be chewed

$$Y=1.5860-0.0005X_1+0.0170X_2-0.0266X_3$$

• Object 2 to be chewed

$$Y=2.9295-0.0011X_1+0.0270X_2-0.0467X_3$$

• Object 3 to be chewed

$$Y=2.5509-0.0007X_1+0.0278X_2-0.0433X_3$$

From Table 1, each adjusted $R^2$ was within a range of from 0.828 to 0.906 and each P-value (<0.05) was also statistically significant, and hence the regression equation for each object to be chewed had high accuracy.

It is possible to evaluate, based on the standard partial regression coefficient β, the magnitudes of the influences of the three explanatory variables (compression force, compression frequency, and number of times of compression) on the progress of the crushing of the object to be chewed (corresponding to the average particle volume of the object to be chewed that has been subjected to the iterative compression process). That is, the explanatory variable with the standard partial regression coefficient β having a larger absolute value can be evaluated as exerting a larger influence on the progress of the crushing of the object to be chewed.

Unexpectedly, in the object 1 to be chewed, the object 2 to be chewed, and the object 3 to be chewed, the compression force exhibited the standard partial regression coefficient β having a smaller absolute value than in the cases of the compression frequency and the number of times of compression, and exerted a smaller influence on the progress of the crushing of the object to be chewed.

The object 1 to be chewed had the smallest absolute value of the standard partial regression coefficient β for the number of times of compression among the objects to be chewed that were subjected to the test.

The object 2 to be chewed had the largest absolute values of the standard partial regression coefficients β for the compression force and the number of times of compression and the smallest absolute value of β for the compression frequency among the objects to be chewed that were subjected to the test.

The object 3 to be chewed had the smallest absolute value of the standard partial regression coefficient β for the compression force and the largest absolute value of the standard partial regression coefficient β for the compression frequency among the objects to be chewed that were subjected to the test.

As described above, the characteristics of the objects to be chewed were successfully evaluated based on, for example, differences in explanatory variables exerting large influences on the progress of the crushing. It was also found that it is effective to develop an object to be chewed based on such evaluation. Further, for example, the characteristics obtained by the regression analysis can be an index for developing an object to be chewed that serves to evaluate a specific eating ability (for example, the occlusal force).

(Example 2)

The objects 1 to 3 to be chewed, which are the same as those in Example 1, and an object 4 to be chewed ("Umeboshi Up" manufactured by Meiji Co., Ltd.) were subjected to the evaluation by the object-to-be-chewed evaluation method.

The processing device for the iterative compression process is the same as that in Example 1.

For each of the objects 1 to 4 to be chewed, the iterative compression process was carried out four times by varying a saliva addition flow rate as the operation condition (process condition of the iterative compression process) of the processing device. Specifically, the saliva addition flow rates were set to 1 ml/min, 2 ml/min, 4 ml/min, and 6 ml/min in the respective processes. In those iterative compression processes, other process conditions were kept constant (compression force: 400 N and compression frequency: 60 times/min). However, the number of times of compression was not required to be constant, which is as described below. At the start of each iterative compression process, a new object to be chewed was subjected to the process.

In each iterative compression process, the time point at which an average of the maximum values of the measurement values of the force for the consecutive two times of compression among the maximum values for the respective times of compression fell below 320 N was determined as the end point of the iterative compression process (therefore, the number of times of compression was not constant but dependent on the end point). Then, the impulse from the start to the end point of the iterative compression process was obtained. The impulse is a parameter that corresponds to the chewiness.

The simple linear regression analysis was performed through use of a logarithmic value of the saliva addition flow rate as the explanatory variable and a logarithmic value of the impulse as the response variable.

For the simple linear regression analysis, "Excel 2016" manufactured by Microsoft Corporation was used.

The regression coefficient was obtained by the simple linear regression analysis.

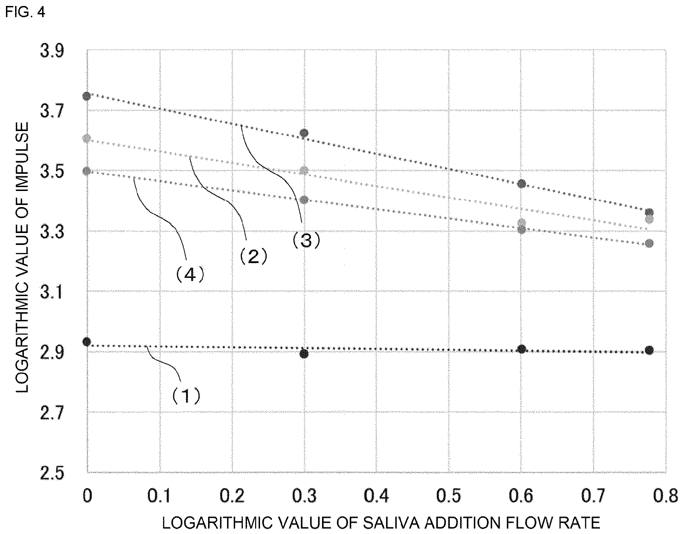

Results of the above are shown in Table 2 and FIG. 4. FIG. 4 is a graph having a horizontal axis representing the logarithmic value of the saliva addition flow rate and a vertical axis representing the logarithmic value of the impulse. In FIG. 4, (1) to (4) correspond to the objects 1 to 4 to be chewed, respectively.

[Table 2]

|  | Regression coefficient |
|---|---|
| Object 1 to be chewed | -0.029 |
| Object 2 to be chewed | -0.380 |
| Object 3 to be chewed | -0.500 |

(continued)

|  | Regression coefficient |
|---|---|
| Object 4 to be chewed | -0.311 |

The regression equations obtained for the objects 1 to 4 to be chewed are as follows. In the following equations, Y represents the logarithmic value of the impulse (N·s), and X represents the logarithmic value of the saliva addition flow rate (ml/min).

- Object 1 to be chewed

$$Y=2.920-0.029X$$

- Object 2 to be chewed

$$Y=3.600-0.380X$$

- Object 3 to be chewed

$$Y=3.755-0.500X$$

- Object 4 to be chewed

$$Y=3.496-0.311X$$

Table 2 and FIG. 4 reveal that the magnitude of the effect of the saliva addition flow rate on the chewiness (impulse) can be evaluated based on the regression coefficient.

The object 1 to be chewed had the smallest absolute value of the regression coefficient, and even when the saliva flow rate changes, an influence on the chewiness is small. This suggests that even when ease of saliva production in the mouth changes, the chewiness is felt in a relatively constant way.

The object 3 to be chewed had the largest absolute value of the regression coefficient. This suggests that when the ease of saliva production in the mouth changes, the chewiness may be felt in a different way as well.

(Example 3)

An existing object to be chewed (referred to also as "reference product"; in this case, "Fruit Juice Gummy Grape" manufactured by Meiji Co., Ltd. was used) and a prototype A were prepared, the prototype A having been obtained by improving the reference product as the object to be chewed for the eating ability test that serves to test a specific ability (in this case, the saliva flow rate) of the eater.

The same processing device as the processing device 1 illustrated in FIG. 1 was used to process each of the reference product and the prototype A with the number of times of compression of 30 times, at a compression frequency of from 40 times/min to 80 times/min, at a compression force of from 200 N to 600 N, and with an artificial saliva addition amount of from 2 ml/min to 6 ml/min. In this case, the simple linear regression analysis was performed through use of a natural logarithm of the average particle volume (parameter based on the object to be chewed that had been subjected to the iterative compression process) as the response variable and a natural logarithm of the artificial saliva addition flow rate (process condition parameter based on the saliva addition amount) as the explanatory variable. As a result, the following regression coefficients were obtained.

[Table 3]

|  | Reference product | Prototype A |
|---|---|---|
| Regression coefficient | -0.91 | -1.14 |

Assuming that the regression coefficient of the reference product was 1, the regression coefficient of the prototype A was 1.25. It was thus considered that the prototype A had been improved to have quality more susceptible to the influence of the saliva flow rate than in the case of the reference product.

That is, it was successfully confirmed that, in the prototype A, the influence of a specific process condition parameter (specific eating condition; in this case, the saliva flow rate) on a specific measurement value parameter (in this case, the natural logarithm of the average particle volume) becomes larger by setting the regression coefficient obtained from the regression analysis as the target quality. Therefore, it is understood that the prototype A has been more suitably improved as the object to be chewed for the eating ability test that serves to test a specific ability (in this case, the saliva flow rate) of the eater.

(Example 4)

An existing object to be chewed (referred to also as the "reference product"; in this case, "Fruit Juice Gummy Grape" manufactured by Meiji Co., Ltd. was used) and a prototype B were prepared, the prototype B having been designed to be able to be eaten in the same manner with various occlusal forces.

The same processing device as the processing device 1 illustrated in FIG. 1 was used to process each of the reference product and the prototype B with the number of times of compression of 30 times, at a compression frequency of from 40 times/min to 80 times/min, at a compression force of from 200 N to 600 N, and with an artificial saliva addition amount of from 2 ml/min to 6 ml/min. In this case, the multiple regression analysis was performed through use of the natural logarithm of the average particle volume (parameter based on the object to be chewed that had been subjected to the iterative compression process) as the response variable and the compression frequency, the compression force, and the artificial saliva addition flow rate (process condition parameters) as the explanatory variables. As a result, the following standard partial regression coefficients $\beta$ were obtained.

[Table 4]

| | | Reference product | Prototype B |
|---|---|---|---|
| Standard partial regression coefficient $\beta$ | Compression frequency | 0.73 | 0.73 |
| | Compression force | -0.40 | -0.28 |
| | Artificial saliva addition flow rate | -0.47 | -0.52 |

Table 4 reveals that, compared to the reference product, the prototype B is equally influenced by the compression speed, is less influenced by the compression force, and is more influenced by the artificial saliva flow rate.

It was thus found that the ease of eating of the prototype B is less liable to be influenced by the specific eating ability (which is the occlusal force in this case, and the occlusal force corresponds to the compression force) of the eater, and with the standard partial regression coefficient being used as the index, it was successfully evaluated whether or not an improvement had been made in accordance with a design intent.

Some embodiments and/or Examples of the present invention have been described above in detail. However, a person skilled in the art can easily make a large number of modifications to those exemplary embodiments and/or Examples without substantially departing from the novel teachings and effects of the present invention. Therefore, such a large number of modifications are within the scope of the present invention.

The contents of all the documents cited herein and of the application from which this application claims priority under the Paris Convention are incorporated by reference herein in their entirety.

Reference Signs List

1: processing device
10: upper plunger
11: upper occlusal portion
12: sensor
20: lower plunger
21: lower occlusal portion
22: protective portion
30: drive unit
40: measurement control unit
50: artificial saliva supply unit
51: inflow tube
AX: rotation axis

LR: reciprocating linear motion
RR: reciprocating rotational motion

**Claims**

1.  A method of evaluating an object to be chewed, the method comprising:

    performing an iterative compression process on the object to be chewed; and
    performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the object to be chewed that has been subjected to the iterative compression process.

2.  The method according to claim 1,

    wherein the iterative compression process is performed through use of a processing device configured to be able to iteratively compress the object to be chewed, and
    wherein the one or more process conditions comprise an operation condition of the processing device.

3.  The method according to claim 1 or 2, wherein the one or more process condition parameters include a process condition parameter based on one or more selected from the group consisting of a compression force, a compression frequency, a compression speed, the number of times of compression, a rotation speed, a rotation angle, an artificial saliva addition amount, an artificial saliva composition, artificial saliva flow characteristics, a temperature, a clearance at a time of compression, a processing amount, and a compression plunger shape.

4.  The method according to claim 1 or 2, wherein the measurement parameter is based on one or more selected from the group consisting of: a load, an impulse, energy, an adhesion force, a torque, a displacement, and a strain that have been applied during the iterative compression process to the object to be chewed, a measurement value obtained by an acoustic sensor, a measurement value obtained by an olfactory sensor, a measurement value obtained by an optical sensor, a measurement value obtained by an electrochemical sensor, and changes over time of the measurement values; and a particle size distribution, hardness, adhesiveness, cohesiveness, a friction coefficient, a viscosity, and a moisture content of the object to be chewed that has been subjected to the iterative compression process, a measurement value obtained by the olfactory sensor, a measurement value obtained by the optical sensor, and a measurement value obtained by the electrochemical sensor.

5.  The method according to claim 1 or 2, wherein the object to be chewed comprises a solid matter or a semi-solid matter.

6.  The method according to claim 1 or 2, wherein the object to be chewed comprises a gummy candy.

7.  The method according to claim 1 or 2, wherein a regression equation obtained by the regression analysis is as follows:

    $$Y=B_0+B_1X_1+B_2X_2+...+B_nX_n$$

    where Y represents the response variable, $B_0$ represents a constant, $B_1$ to $B_n$ each represent a regression coefficient, $X_1$ to $X_n$ each represent one of the explanatory variables, and "n" represents an integer of 1 or more.

8.  An object to be chewed, which is formed with an evaluation result obtained by the method of claim 1 or 2 being set as target quality.

9.  A manufacturing method for an object to be chewed, the manufacturing method comprising the method of claim 1 or 2.

10. A system for evaluating an object to be chewed, the system comprising:

    means for performing an iterative compression process on the object to be chewed; and

means for performing a regression analysis through use of, as explanatory variables, one or more process condition parameters based on one or more process conditions of the iterative compression process and, as a response variable, a measurement parameter based on one or more measurement values selected from the group consisting of a measurement value obtained from the object to be chewed that is being subjected to the iterative compression process and a measurement value obtained from the object to be chewed that has been subjected to the iterative compression process.

11. A program for causing a computer to operate each means of claim 10.

12. A computer-readable recording medium having recorded thereon the program of claim 11.

13. The recording medium according to claim 12, wherein the recording medium comprises a server on a network.

FIG. 1

FIG. 2

FIG. 3

FIG. 4

## INTERNATIONAL SEARCH REPORT

| International application No. |
|---|
| **PCT/JP2024/012951** |

**A. CLASSIFICATION OF SUBJECT MATTER**

***G01N 3/34***(2006.01)i; ***G01N 33/02***(2006.01)i
FI: G01N3/34 M; G01N33/02

According to International Patent Classification (IPC) or to both national classification and IPC

**B. FIELDS SEARCHED**

Minimum documentation searched (classification system followed by classification symbols)

G01N3/34; G01N33/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2021/033583 A1 (NATIONAL UNIVERSITY CORPORATION KOBE UNIVERSITY) 25 February 2021 (2021-02-25) entire text | 1-13 |
| A | JP 2022-187490 A (UNIV IWATE) 19 December 2022 (2022-12-19) entire text | 1-13 |
| A | JP 2010-539504 A (UNIVERSITE D'AUVERGNE CLERMONT 1) 16 December 2010 (2010-12-16) entire text | 1-13 |
| A | US 2022/0003734 A1 (KNU-INDUSTRY COOPERATION FOUNDATION) 06 January 2022 (2022-01-06) entire text | 1-13 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/012951**

| Patent document cited in search report | Publication date (day/month/year) | Patent family member(s) | Publication date (day/month/year) |
|---|---|---|---|
| WO 2021/033583 A1 | 25 February 2021 | (Family: none) | |
| JP 2022-187490 A | 19 December 2022 | (Family: none) | |
| JP 2010-539504 A | 16 December 2010 | US 2014/0272875 A1 entire text | |
| US 2022/0003734 A1 | 06 January 2022 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001133374 A
- JP 2017026484 A
- JP 2021085834 A
- JP 2020134526 A
- JP 2022066154 A
- WO 2022250167 A1